# EUROPÄISCHE PATENTSCHRIFT

(11) **EP 1 180 356 B1**
(45) Veröffentlichungstag und Bekanntmachung des Hinweises auf die Patenterteilung: **02.04.2008**
(21) Anmeldenummer: 01118397.7
(22) Anmeldetag: 28.07.2001
(51) Int. Cl.: A61K 8/88, C11D 3/37

(54) **Konditionierendes Haarwaschmittel**
Conditioning shampoo
Shampooing conditionneur

(30) Priorität: 18.08.2000 DE 10040514
(43) Veröffentlichungstag der Anmeldung: 20.02.2002
(73) Patentinhaber: KPSS-Kao Professional Salon Services GmbH, 64297 Darmstadt (DE)
(72) Erfinder: Fath, Bettina, 69469 Weinheim (DE); Dubowoj, Polina, Aree Place Condominium Room 801, Klongton, Klongtoey, Bangkok 10110 (TH)

(56) Entgegenhaltungen:
- EP-A- 0 745 379
- EP-A- 0 846 462
- WO-A-95/34271
- FR-A- 2 555 441

## Beschreibung

Die Erfindung betrifft ein konditionierendes Haarwaschmittel mit verbesserten Eigenschaften.

Von einem guten Haarwaschmittel erwartet der Verbraucher vor allem eine zufriedenstellende Reinigungswirkung, eine gute Schaumentwicklung bei der Anwendung, sparsamen Verbrauch, gute Haut- und Schleimhautverträglichkeit ohne jedwede hautreizende oder allergisierende Wirkung und zumeist auch einen haarkonditionierenden Effekt

WO 95/34271 A1 beschreibt ein festigendes Haarreinigungsmittel enthaltend ein anionisches Tensid, mindestens ein kationisches Polymer, mindestens ein neutralisiertes anionisches Polymer und einen dispergierten Feststoff mit einer mittleren Korngrösse von 1 bis 200 Mikrometer.

Es sind zahlreiche Shampoos bekannt, die sich bemühen, diesen Anforderungen gerecht zu werden, ohne dies allerdings immer zu erreichen. Die Erfindung stellt nun ein Shampoo zur Verfügung, das die obengenannten Voraussetzungen erfüllt
Das erfindungsgemäße Haarwaschmittel wird dadurch erhalten, daß einer wäßrigen Grundlage, enthaltend 5 bis 50 Gew.-%, bezogen auf die Gesamtzusammensetzung, mindestens eines anionischen, amphoteren, zwitterionischen und/oder nichtionischen Tensids, wobei das Mittel mindestens ein anionisches Tensid und mindestens ein amphoterisches Tensid enthält mindestens ein pulverförmiges Polyamid mit einem Teilchendurchmesser von mindestens 80% im Bereich von 1 bis 20 Mikron und 0,25 bis 15 Gew.-% mindestens eines haarkonditionierenden Wirkstoffs zugesetzt wird.

Geeignete pulverförmige Polyamide, die in den erfindungsgemäßen Haarwaschmitteln vorzugsweise in einer Menge von etwa 0,1 bis etwa 10, insbesondere etwa 0,25 bis etwa 7,5, vor allem 0,5 bis 5 Gew.-%, bezogen auf die Gesamtzusammensetzung des Mittels, eingesetzt werden, sind an sich bekannt, beispielsweise als "Polyamide 6° oder "12" unter der Bezeichnung "Nylon". 80% der Teilchen weisen dabei einen Teilchendurchmesser im Bereich von 1 bis 20, vorzugsweise etwa 2,5 bis 17, insbesondere etwa 5 bis 15 Mikron auf.

Weitere Handelsprodukte sind "Orgasol^{R}1002", "2002" und "4000".

Die erfindungsgemäßen Shampoo-Zusammensetzungen enthalten mindestens ein anionisches Tensid, in einer Menge von vorzugsweise 10 bis 25 Gew.-% der Zusammensetzung.

Geeignete anionaktive Tenside im Rahmen der Erfindung sind solche vom Sulfat-, Sulfonat-, Carboxylat- und Alkylphosphat-Typ, vor allem natürlich diejenigen, die in Shampoos üblicherweise zum Einsatz gelangen, beispielsweise die bekannten C₁₀-C₁₈-Alkylsulfate und insbesondere die entsprechenden Ethersulfate, beispeilsweise C₁₂-C₁₄-Alkylethersulfat, Laurylethersulfat, insbesondere mit 1 bis 4 Ethylenoxidgruppen im Molekül, weiterhin Monoglycerid(ether)sulfate Fettsäureamidsulfate, die durch Ethoxylierung und anschließende Sulfatierung von Fettsäurealkanolamiden erhalten werden, und deren Alkalisalze sowie Salze langkettige Mono- und Dialkylphosphate, die milde, hautverträgliche Detergentien darstellen.

Im Rahmen der Erfindung weiterhin geeignete anionische Tenside sind α-Olefinsulfonate bzw. deren Salze und insbesondere Alkalisalze von Sulfobemsteinsäurehalbestern, beispielsweise das Dinatriumsalz des Monooctylsulfosuccinats und Alkalisalze langkettiger Monoalkylethoxysulfosuccinate. Geeignete Tenside vom Carboxylat-Typ sind Alkylpolyethercarbonsäuren und deren Salze der Formel

R-(C₂H₄O)ₙ-O-CH₂COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 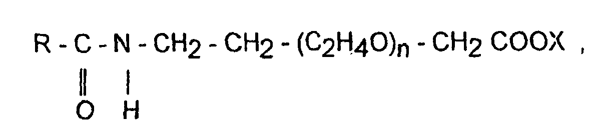 worin R und X die vorstehend angegebene Bedeutung haben, und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO^{®} " und "AKYPO-SOFT^{®}".

Auch C₈-C₂₀-Acylisethionate können, allein oder im Gemisch mit anderen Tensiden, eingesetzt werden, ebenso Sulfofettsäuren und deren Ester.
Es können auch Mischungen aus mehreren anionischen Tensiden eingesetzt werden, beispielsweise ein Gemisch aus einem α-Olefinsulfonat und einem Sulfosuccinat, vorzugsweise im Verhältnis von 1 : 3 bis 3 : 1, oder einem Ethersulfat und einer Polyethercarbonsäure oder Alkylamidoethercarbonsäure.

Eine Übersicht über die in flüssigen Körperreinigungsmitteln zum Einsatz gelangenden anionaktiven Tenside findet sich im übrigen in der Monographie von K.Schrader, "Grundlagen und Rezepturen der Kosmetika", 2. Aufl. (1989, Hüthig Buchverlag), S. 595-600 und S. 683 bis 691.
Weitere geeignete anionische Tenside sind auch C₈-C₂₂-Acylaminocarbonsäuren bzw. deren wasserlösliche Salze, vorzugsweise in einer Menge von 0,5 bis 10, insbesondere 1 bis 7,5 Gew.-%, berechnet auf die Gesamtzusammensetzung. Besonders bevorzugt sind N-Lauroylglutamat, insbesondere als Natriumsalz, sowie beispielsweise N-Lauroylsarcosinat, N-C₁₂-C₁₈-Acylasparaginsäure, N-Myristoylsarcosinat, N-Oleoylsarcosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze, vorzugsweise im Gemisch mit den obengenannten anionaktiven Tensiden.

Der besonders bevorzugte Mengenbereich an anionischen Tensiden in den erfindungsgemäßen flüssigen Shampoos liegt zwischen etwa 5 und etwa 35 Gew.-%, insbesondere bei etwa 7,5 bis etwa 25 Gew.-%, besonders bevorzugt bei etwa 10 bis etwa 20 Gew.-%, berechnet auf die Gesamtzusammensetzung des Mittels.

Weitere geeignete Tenside in den erfindungsgemäßen Haarwaschmitteln sind nichtionische Tenside, vorzugsweise im Gemisch mit anionaktiven und/oder zwitterionischen bzw. amphoteren Tensiden.
Diese sind bei Schrader, I.c., auf den Seiten 600-601 und S. 694-695 beschrieben. Geeignet sind insbesondere Alkylpolyglucoside mit der allgemeinen Formel

R-O-(R¹O)ₙ-Zₓ,

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 5 bedeuten.
Diese Alkylpolyglucoside sind in letzter Zeit insbesondere als ausgezeichnete hautverträgliche schaumverbessernde Mittel in flüssigen Wasch- und Körperreinigungsmitteln bekannt geworden und sind in einer Menge von 1 bis 20, insbesondere 2,5 bis 10 Gew.-%, der Gesamtzusammensetzung enthalten.
Gemische aus anionaktiven Tensiden und Alkylpolyglucosiden sowie deren Verwendung in flüssigen Körperreinigungsmitteln sind an sich bereits bekannt, beispielsweise aus der EP-A 70 074. Die dort beschriebenen Alkylpolyglucoside sind prinzipiell auch im Rahmen der vorliegenden Erfindung geeignet; ebenso die aus der EP-A 358 216 bekannten Gemische aus Sulfosuccinaten und Alkylpolyglucosiden.

Weitere nichtionische Tensidbestandteile sind beispielsweise langkettige Fettsäuremono- und -dialkanolamide, wie Cocosfettsäuremonoethanolamid und Myristinfettsäuremonoethanolamid, die auch als Schaumverstärker eingesetzt werden können.

Andere zusätzlich mitverwendbare nichtionische Tenside sind z.B. die verschiedenen Sorbitanester, wie Polyethylenglykolsorbitanstearinsäureester, Fettsäurepolyglykol-ester oder auch Mischkondensate aus Ethylenoxid und Propylenoxid, wie sie beispielsweise unter der Handelsbezeichnung "Pluronics" im Verkehr sind.
Weitere zusätzlich einsetzbare Tenside sind Aminoxide in einer Menge von etwa 0,25 bis etwa 5, vorzugsweise etwa 0,5 bis etwa 3,5 Gew.-%, berechnet auf die Gesamtzusammensetzung des Mittels.
Solche Aminoxide gehören seit langem zum Stand der Technik, beispielsweise C₁₂-C₁₈-Alkyldimethylaminnxide wie Lauryldimethylaminoxid, C₁₂-C₁₈-Alkylamidopropyl- oder -ethylaminoxide, C₁₂-C₁₈-Alkyldi(hydroxyethyl)- oder -(hydroxypropyl)aminoxide, oder auch Aminoxide mit Ethylenoxid- und /oder Propylenoxidgruppen in der Alkylkette.
Aminoxide sind beispielsweise unter den Bezeichnungen "Ammonyx^{®}", "Aromox^{®}" oder "Genaminox^{®}" im Handel.
Die erfindungsgemäßen Zusammensetzungen enthalten mindestens ein amphoterisches bzw. zwitterionisches Tensid, beispielsweise in einer Menge von etwa 0,5 bis etwa 10, vorzugsweise von etwa 1 bis etwa 7,5 Gew.-%, bezogen auf die Gesamtzusammensetzung.
Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.

Im einzelnen können Betaine der Struktur 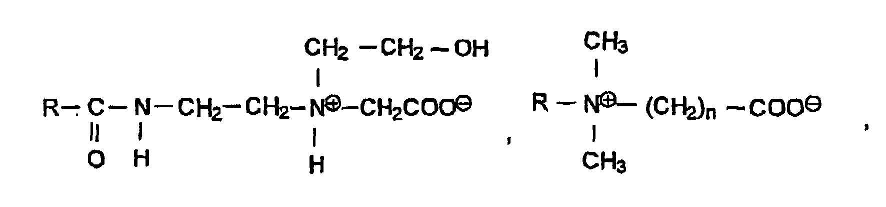 und wobei R eine C₈-C₁₈-Alkylgruppe und n 1 bis 3 bedeuten,
Sulfobetaine der Struktur 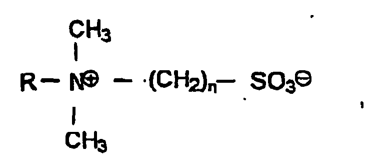 wobei R eine C₈-C₁₈-Alkylgruppe und n 1 bis 3 bedeuten,
und Amidoalkylbetaine der Struktur 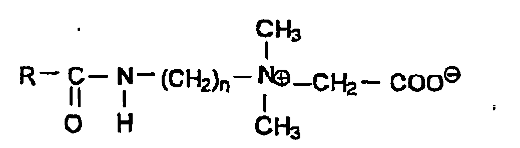 wobei R eine C₈-C₁₈-Alkylgruppe und n 1 bis 3 bedeuten, verwendet werden

Die erfindungsgemäßen Shampoos enthalten mindestens einen haarkonditionierenden Wirkstoff wie Eiweißhydrolysate und Polypeptide, z.B., Keratinhydrolysate, Kollagenhydrolysate vom Typ "Nutrilan^{®}" oder Elastinhydrolysate sowie insbesondere auch pflanzliche, gegebenenfalls kationisierte Eiweißhydrolysate, z.B. "Gluadin^{®}", enthalten.

Weitere bevorzugte mögliche Zusätze sind haarkonditionierende Polymere.
Diese können nichtionische Polymere, vorzugsweise alkohol- und/oder wasserlösliche Vinylpyrrolidon-Polymere wie ein Vinylpyrrolidon-Homopolymerisat oder -Copolymerisat, insbesondere mit Vinylacetat, sein.
Geeignete Vinylpyrrolidon-Polymere sind z.B. die unter dem Handelsnamen "Luviskol^{®}" bekannten Produkte, beispielsweise die Homopolymerisate "Luviskol^{®} K 30, K 60 und K 90" sowie die wasser- bzw. alkohollöslichen Copolymerisate aus Vinylpyrrolidon und Vinylacetat, die unter dem Handelsnamen "Luviskol^{®} VA 55 bzw. VA 64" von der BASF AG vertrieben werden.
Weitere geeignete nichtionische Polymere sind Vinylpyrrolidon/Vinylacetat/Vinylpropionat-Copolymere wie "Luviskol^{®} VAP 343", Vinylpyrrolidont(Meth)Acrylsäureester-Copolymere sowie Chitosan-Derivate. Ihr Anteil in den erfindungsgemäßen Haarwaschmitteln liegt, wenn vorhanden, zwischen etwa 0,05 und etwa 5, vorzugsweise 0,1 und 2,5, insbesondere etwa 0,15 bis 1,5 Gew.-%, bezogen auf die Gesamtzusammensetzung des Mittels.

Anstelle oder zusätzlich zu den nichtionischen Polymeren können als haarkonditionierende Polymere auch kationische und/oder anionische und/oder amphotere Polymere in den genannten Mengen eingesetzt werden.

Bevorzugte haarkonditionierende kationische Polymere sind die altbekannten quaternären Cellulosederivate des Typs "Polymer JR" sowie quaternisierte Homo- und Copolymere des Dimethyldiallylammoniumchlorids, wie sie unter dem Handelsnamen "Merquat^{®}" im Handel sind, quaternäre Vinylpyrrolidon-Copolymere, insbesondere mit Dialkylaminoalkyl(meth)acrylaten, wie sie unter dem Namen "Gafquat^{®}" bekannt sind, Copolymerisate aus Vinylpyrrolidon und Vinylimidazoliniummethochlorid, die unter dem Handelsnamen "Luviquat^{®}" angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniumverbindungen der in der US-PS 4 157 388 beschriebenen Harnstoff-Struktur, die unter dem Handelsnamen "Mirapol® A 15" im Handel sind.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Als amphotere Polymere, die allein oder im Gemisch mit mindestens einem weiteren kationischen, nichtionischen oder anionischen Polymeren zum Einsatz gelangen, seien insbesondere Copolymerisate aus N-Octylacrylamid, (Meth)Acrylsäure und tert-Butylaminoethylmethacrylat vom Typ "Amphomer®"; Copolymerisate aus Methacryloylethylbetain und Alkylmethacrylaten vom Typ "Yukaformer®", z. B. das Butylmethacrylat-Copolymere "Yukaformer® Am75"; Copolymerisate aus Carboxylgruppen und Sulfongruppen enthaltenden Monomeren, z. B. (Meth)Acrylsäure und Itaconsäure, mit basischen Gruppen, insbesondere Aminogruppen, enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert-Butylaminoethylmethacrylat und Acrylsäure sowie die aus der US A 3,927,199 bekannten Copolymeren genannt.

Geeignete anionische Polymere sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez® AN oder ES" vertrieben werden. Diese Polymeren können auch teilverestert sein, beispielsweise "Gantrez® ES 225", der Ethylester eines Ethylvinylethers/Maleinsäure-Copolymers, oder der Butyl- oder Isobutylester desselben.
Weitere geeignete anionische Polymere sind insbesondere Vinylacetat/Crotonsäure- oder Vinylacetat/Vinylneodecanoat/Crotonsäure-Copolymere des Typs "Resyn®"; Natriumacrylat/Vinylalkohol-Copolymere des Typs "Hydagen® F", Natriumpolystyrolsulfonat, z.B. "Flexan® 130"; Ethylacrylat/Acrylsäure/N-tert-Butylacrylamid-Copolymere des Typs "Ultrahold®"; Vinylpyrrolidon/Vinylacetat/Itaconsäure-Copolymere, Acrylsäure/Acrylamid-Copolymere bzw. Natriumsalze derselben vom Typ "Reten®"; etc.

Die erfindungsgemäßen Shampoos können selbstverständlich alle üblichen, in solchen Mitteln zum Einsatz gelangenden Stoffe enthalten.

Als solche seien beispielhaft Komplexbildner, Farbstoffe, Konservierungsmittel, pH-Regler, Viskositätsregler wie anorganische Salze, soweit sie nicht ohnehin in den Tensid-Ausgangsmischungen enthalten sind, Duftstoffe, Perlglanzmittel, Verdickungsmittel, Feuchthaltemittel, pflanzliche und tierische Öle, etc. genannt.

Geeignete pflanzliche und tierische Öle sowie synthetische Fettsäureester, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven-bzw. Sojaöl, Lanolin und dessen Derivate, ebenso Mineralöle wie Paraffinöl und Vaseline, die ebenfalls haarkonditionierende Eigenschaften besitzen. Synthetische Öle und Wachse sind beispielsweise Silikonöle, Polyethylengykole, etc. Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, - palmitat, -stearat und -isostearat, Oleyloleat, Isocetylstearat, Hexyllaurat, Dibutyladipat, Dioctyladipat, Myristylmyristat, Oleylerucat, Polyethlenglykol- und Polyglycerylfettsäureester wie PEG-7-glycerylcocoat, Cetylpalmitat, etc.
Diese hydrophoben Komponenten sind in der erfindungsgemäßen Zusammensetzung vorzugsweise in einer Gesamtmenge von etwa 0,5 bis etwa 10, insbesondere etwa 1 bis 7,5, vor allem etwa 1,5 bis 5 Gew.-%, berechnet auf die Gesamtzusammensetzung, enthalten.

Eine Auflistung solcher Zusatzstoffe findet sich ebenfalls bei Schrader, l.c., auf S.695 bis 722.

Ein weiterer bevorzugter Bestandteil ist Ethoxydiglykol, vorzugsweise in einer Menge von 0,1 bis 5 Gew.-% des erfindungsgemäßen Shampoos.

Schließlich können auch noch bekannte Polysiloxane als konditionierende Mittel in den erfindungsgemäßen flüssigen Haarwaschmitteln mitverwendet werden. Deren bevorzugter Anteil liegt dabei etwa zwischen 0,5 und etwa 5, insbesondere 1 bis 3 Gew.-% der Gesamtzusammensetzung. Geeignet sind sowohl leichtflüchtige als auch schwerflüchtige cyclische oder lineare Polysiloxane, beispielsweise die unter dem Trivialnamen "Dimethicone" bzw. "Phenyldimethicone" sowie "Cyclomethicone" bekannten Silikonöle.
Geeignet sind beispielsweise auch die in der EP-A 398 177 beschriebenen Silikonderivate, die dort in Kombination mit Alkylpolyglucosiden in flüssigen Detergens-Zusammensetzungen eingesetzt werden.

Gemäß einer bevorzugten Ausführungsform der Erfindung können die erfindungsgemäßen Shampoos auch Farbstoffe zur direkten oder oxidativen Färbung von Haaren enthalten, also sogenannte Tönungs- oder Färbeshampoos. Besonders geeignet sind direktziehende Farbstoffe enthaltende Tönungsshampoos. Es wurde nämlich festgestellt, daß solche Shampoos, die Polyamidpulver und insbesondere kationische direktziehende Farbstoffe enthalten, eine besonders brillante, glänzende und stabile Haarfärbung gegenüber bekannten Tönungsshampoos auf Basis konventioneller Shampoos ergeben.

Der pH-Wert der erfindungsgemäß eingesetzten Shampoos liegt im üblichen Bereich zwischen etwa 5 und 8,5; für Spezialprodukte kann er auch unterhalb 5 eingestellt werden.

Die Viskosität liegt im üblichen Bereich zwischen etwa 500 und etwa 50000 mPa.s bei 20°C, vorzugsweise etwa 1000 bis etwa 25000, insbesondere 2500 bis 10000 mPa.s bei 20°C, gemessen nach Brookfield oder Höppler bei einer Scherspannung von 10 sec⁻¹.

Diese Zusammensetzungen können auch als Aerosol-Shampoos mit den üblichen Treibmitteln in Aluminiummonoblockdosen als auch Weißblechdosen, vorzugsweise mit Innenbeschichtung, z.B. auf Epoxyharz-Basis, versehen, als auch Kunststoffdosen abgepackt werden.
Die Herstellung der Produkte erfolgt durch Zusammenrühren der einzelnen Komponenten in Wasser, wobei auch Vormischungen verschiedener Bestandteile verwendet werden können.

Die folgenden Beispiele dienen der Illustration der Erfindung.

### Beispiele

| Bestandteile | Nr. 1 | Nr. 2 | Nr.3 | Nr.4 |
|---|---|---|---|---|
| Natriumalkylethersulfat, (~2,5 EO) | 10,0 | 8,5 | 3,5 | 8,0 |
| Natriumlauroylglutamat | 6,0 | - | - | - |
| Cocoamphoacetat | - | 3,0 | 2,0 | 2,5 |
| Cocoamidopropylbetain | 2,0 | - | - | - |
| PEG-3-distearat | 2,0 | - | - | 1,5 |
| C₁₂-C₁₄-Alkylpolyglucosid (P.D. ~1,5) | - | 2,0 | 3,0 | - |
| Polyquaternium-7 | 1,0 | - | - | - |
| AcrylateslAminoacrylates / C₁₀-C₃₀-Alkyl PEG20-Itaconate Copolymer | - | 2, 5 | - | 0,8 |
| Triethanolamin | - | - | 0,3 | - |
| Acrylates C₁₀-C₃₀ Alkyl Acrylate Copolymer | - | - | 0,6 | - |
| PEG-60-hydriertes Ricinusöl | - | 0,4 | 0,5 | 0,5 |
| Natriumchlorid | 1,2 | - | - | - |
| Nylon-12 (mittl. Teilchendurchmesser: 90% zwischen 3,5 und 12,5 µm) | 0,6 | 0,5 | 0,7 | 0,4 |
| Mandelöl | - | - | 0,2 | - |
| Dimethicone Copolymer | - | - | 0,4 | - |
| Parfum | 0,4 | 0,4 | 0,3 | 0,3 |
| Citronensäure | 0,1 | 1,0 | - | 0,5 |
| Panthenol | 0,5 | - | 0,3 | - |
| Tocopherylacetat | 0,1 | - | 0,2 | - |
| Natriumbenzoat | 0,5 | 0,5 | 0,5 | 0,5 |
| Wasser ad | 100,0 | 100,0 | 100,0 | 100,0 |

Diese Shampoo-Zusammensetzungen zeigten ein ausgezeichnetes Schaumvermögen mit cremigem Schaum (überraschenderweise auch die Zusammensetzung Nr. 3, die nur wenig anionisches Tensid enthielt) und ließen sich leicht auf dem Haar verteilen.
Bei dreimaliger Anwendung der Shampoos von jeweils 10 Personen traten keinerlei allergischen oder hautsensibilisierenden Effekte auf.
Überraschenderweise ergab sich eine eindeutige Präferenz der Zusammensetzungen nach den Beispielen gegenüber gleichen Zusammensetzungen, die kein Polyamidpulver enthielten, hinsichtlich des Glanzes, der Kämmbarkeit, der Glätte, der Lockerheit, der Sprungkraft und des Volumens der Haare im Doppelblindversuch an jeweils 10 Personen.

## Patentansprüche

1. Haarwaschmittel auf wäßriger Basis, enthaltend 5 bis 50 Gew.-%, berechnet auf die Gesamtzusammensetzung, mindestens eines anionischen, nichtionischen, amphoteren und/oder zwitterionischen Tensids, wobei das Mittel mindestens ein anionisches Tensid und mindestens ein amphoterisches Tensid enthält, **dadurch gekennzeichnet dass** es mindestens ein pulverförmiges Polyamid mit einem Teilchendurchmesser von mindestens 80% im Bereich von 1 bis 20 Mikron und 0,25 bis 15 Gew.-% mindestens eines haarkonditionierenden Wirkstoffs enthält.

2. Mittel nach Anspruch 1, enthaltend 0,1 bis 10 Gew.-% des pulverförmigen Polyamids.

3. Mittel nach Ansprüche 1 und 2, enthaltend als haarkonditionierenden Wirkstoff eines lipophilen Stoffs und/oder synthetischen oder natürlirchen kationischen, anionischen, nichtionischen, amphoteren und/oder zwitterionischen Polymeren.

## Claims

1. Shampoo composition on aqueous basis, comprising 5 % to 50 % by weight, calculated to the total composition, of at least one anionic, nonionic, amphoteric and/or zwitterionic surfactant, whereby the composition contains at least one anionic and at least one amphoteric surfactant, **characterized in that** it contains at least one pulverulent polyamide, whereby at least 80 % of the particles thereof have a particle diameter in the range of 1 to 20 microns, and 0.25 to 15 % by weight of at least one hair-conditioning agent.

2. Composition according to claim 1, comprising 0.1 % to 10 % by weight, calculated to the total composition, of the pulverulent polyamide.

3. Composition according to claims 1 or 2, comprising as hair-conditioning agent a lipophilic substance and/or synthetic or natural, cationic, anionic, nonionic amphoteric and/or zwitterionic polymer.

## Revendications

1. Agent de lavage capillaire à base aqueuse, contenant 5 à 50% en poids, calculés par rapport à la composition totale, d'au moins un tensioactif anionique, non ionique, amphotère, et/ou zwittérionique, l'agent contenant au moins un tensioactif anionique et au moins un tensioactif amphotère, **caractérisé en ce qu'**il contient au moins un polyamide sous forme de poudre avec un diamètre de particule, à raison d'au moins 80%, dans la plage de 1 à 20 µm et 0,25 à 15% en poids d'au moins une substance active de conditionnement capillaire.

2. Agent selon la revendication 1, contenant 0,1 à 10 % en poids du polyamide sous forme de poudre.

3. Agent selon les revendications 1 et 2, contenant en tant que substance active de conditionnement capillaire, une matière lipophile et/ou des polymères cationiques, anioniques, non ioniques, amphotères et/ou zwittérioniques, synthétiques ou naturels.
